# EUROPEAN PATENT APPLICATION

(11) **EP 3 245 930 A1**
(43) Date of publication of application: **22.11.2017**
(21) Application number: 15878048.6
(22) Date of filing: 28.12.2015
(51) Int. Cl.: A61B 1/00

(54) **OVER TUBE**

(30) Priority: 15.01.2015 JP 2015005544
(71) Applicant: Olympus Corporation, Tokyo 192-8507 (JP)
(72) Inventor: YANAGIHARA, Masaru, Tokyo 192-8507 (JP); KISHI, Kosuke, Tokyo 192-8507 (JP)
(74) Representative: Schicker, Silvia
(86) International application number: PCT/JP2015/086466
(87) International publication number: WO 2016/114097

(57) **Abstract**

An overtube that is capable of being inserted by an endoscope and a treatment tool and is elongated in a direction of a predetermined central axis, the overtube includes a tube part in which a first lumen and a second lumen are formed, the first lumen being capable of being inserted by the endoscope, and the second lumen being capable of being inserted by the treatment tool; and a distal end part in which a distal opening end of the first lumen and a distal opening end of the second lumen are formed apart from each other, wherein the distal end part includes: a distal end surface that forms the distal opening end of the first lumen such that a center of the distal opening end of the first lumen is located at a position apart from the predetermined central axis, and a tapered surface that is inclined with respect to the predetermined central axis such that the tapered surface gradually approaches the first lumen from a proximal side toward a distal side in the direction of the predetermined central axis, at least part of the distal opening end of the second lumen being disposed on the tapered surface.

## Description

### [Technical Field]

The present invention relates to an overtube.

Priority is claimed on Japanese Patent Application No. 2015-005544, filed on January 15, 2015, the content of which is incorporated herein by reference.

### [Background Art]

In the related art, an overtube used for guiding an endoscope, a treatment tool, and the like having an elongated insertion part to a treatment target site in the body is known (for example, refer to Patent Documents 1, 2, and 3).

The overtube is inserted together with the endoscope, the treatment tool, and the like or before inserting the endoscope, the treatment tool, and the like into the inside of the body. For example, an overtube to be inserted into a gastrointestinal tract rides over a fold of the gastrointestinal tract, or passes through a narrow segment of the gastrointestinal tract, and is advanced up to a treatment target site.

### [Citation List]

### [Patent Literature]

[Patent Document 1] Published Japanese Translation No. 2011-525125 of the PCT International Publication
[Patent Document 2] Published Japanese Translation No. 2008-540041 of the PCT International Publication
[Patent Document 3] Japanese Unexamined Patent Application, First Publication No. 2003-210398

### [Summary of Invention]

### [Technical Problem]

Since the overtube includes a plurality of lumens to which a plurality of medical instruments, such as an endoscope and a treatment tool, are attached, the overtube has a large diameter in many cases. In a process in which the overtube rides over the fold of the gastrointestinal tract or passes through the narrow segment of the gastrointestinal tract, a distal end of the overtube contacts the fold or the narrow segment, and the insertion resistance is generated when the overtube is advanced to the treatment target site. In the techniques disclosed in Patent Documents 1, 2, and 3, the larger diameter the overtube has, the insertion resistance becomes significantly larger.

The present invention has been made in consideration of the above-described circumstances, and an object thereof is to provide an overtube with a small insertion resistance.

### [Solution to Problem]

According to a first aspect of the present invention, an overtube that is capable of being inserted by an endoscope and a treatment tool and is elongated in a direction of a predetermined central axis includes: a tube part in which a first lumen and a second lumen are formed, the first lumen being capable of being inserted by the endoscope, and the second lumen being capable of being inserted by the treatment tool; and a distal end part in which a distal opening end of the first lumen and a distal opening end of the second lumen are formed apart from each other. The distal end part includes a distal end surface that forms the distal opening end of the first lumen such that a center of the distal opening end of the first lumen is located at a position apart from the predetermined central axis, and a tapered surface that is inclined with respect to the predetermined central axis such that the tapered surface gradually approaches the first lumen from a proximal side toward a distal side in the direction of the predetermined central axis, at least part of the distal opening end of the second lumen being disposed on the tapered surface.

According to a second aspect of the present invention, the overtube according to the first aspect may further include a first sealing part that is configured to airtightly seal the first lumen and airtightly seal a gap between the first lumen and the endoscope in a state where the endoscope is disposed within the first lumen; and a second sealing part that is configured to airtightly seal the second lumen and airtightly seal a gap between the second lumen and the treatment tool in a state where the treatment tool is disposed within the second lumen.

According to a third aspect of the present invention, in the overtube according to the first aspect or second aspect, the distal end part may include a cover part capable of airtightly sealing the distal end opening of the second lumen and capable of being opened and closed.

According to a fourth aspect of the present invention, in the overtube according to the third aspect, the cover part may be capable of contacting the distal opening end of the second lumen from a distal side thereof and is movably coupled to the distal end part such that the distal opening end of the second lumen is opened by movement of the cover part toward more distal side than the distal opening end of the second lumen.

According to a fifth aspect of the present invention, in the overtube according to any one aspect the first aspect to fourth aspect, the distal opening end of the first lumen may be larger than a proximal opening end of the first lumen.

According to a sixth aspect of the present invention, in the overtube according to any one aspect of the first aspect to fifth aspect, the first lumen may have a small-diameter part which is formed with an inner diameter smaller than an inner diameter of the distal opening end of the first lumen and through which the endoscope is capable of being inserted, at part of the first lumen more proximal than the distal opening end of the first lumen.

According to a seventh aspect of the present invention, in the overtube according to any one aspect of the first aspect to sixth aspect, at least part of the distal opening end of the second lumen may be formed by the tapered surface.

According to an eighth aspect of the present invention, the overtube according to any one aspect the first aspect to seventh aspect may further include a movable mechanism that is configured to move the distal opening end of the second lumen with respect to the distal opening end of the first lumen.

### [Advantageous Effects of Invention]

According to the overtube according to the above respective aspects, the overtube with a small insertion resistance in use can be provided.

### [Brief Description of Drawings]

FIG. 1 is a side view showing a portion of an overtube according to a first embodiment of the invention in a section.
FIG. 2 is a front view of the overtube according to the first embodiment of the invention.
FIG. 3 is a front view showing another configuration example of the overtube according to the first embodiment of the invention.
FIG. 4 is a view for explaining the operation of the overtube according to the first embodiment of the invention.
FIG. 5 is a view for explaining the operation of the overtube according to the first embodiment of the invention.
FIG. 6 is a view for explaining the operation of the overtube according to the first embodiment of the invention.
FIG. 7 is a view for explaining the operation of the overtube according to the first embodiment of the invention.
FIG. 8 is a view for explaining the operation of the overtube according to the first embodiment of the invention.
FIG. 9 is a view for explaining the operation of the overtube according to the first embodiment of the invention.
FIG. 10 is a sectional view showing the configuration of an overtube in a modification example of the first embodiment of the invention.
FIG. 11 is a sectional view showing another configuration example in the above-mentioned modification example of the first embodiment of the invention.
FIG. 12 is a sectional view showing still another configuration example in the above-mentioned modification example of the first embodiment of the invention.
FIG. 13 is a side view showing a portion of an overtube according to a second embodiment of the invention.
FIG. 14 is a front view of the overtube according to the second embodiment of the invention.
FIG. 15 is a sectional view in line A-A of FIG. 14.
FIG. 16 is a sectional view in line B-B of FIG. 14.
FIG. 17 is a side view showing another configuration example of the overtube according to the second embodiment of the invention.
FIG. 18 is a front view showing the above-mentioned other configuration example of the second embodiment of the invention.
FIG. 19 is a side view showing a portion of an overtube according to a third embodiment of the invention.
FIG. 20 is a front view of the overtube according to the third embodiment of the invention.
FIG. 21 is a sectional view in line C-C of FIG. 20.
FIG. 22 is a view for explaining the operation of the overtube according to the third embodiment of the invention.
FIG. 23 is a view for explaining the operation of the overtube according to the third embodiment of the invention.
FIG. 24 is a sectional view showing an overtube according to a fourth embodiment of the invention.

### [Description of Embodiments]

### (First Embodiment)

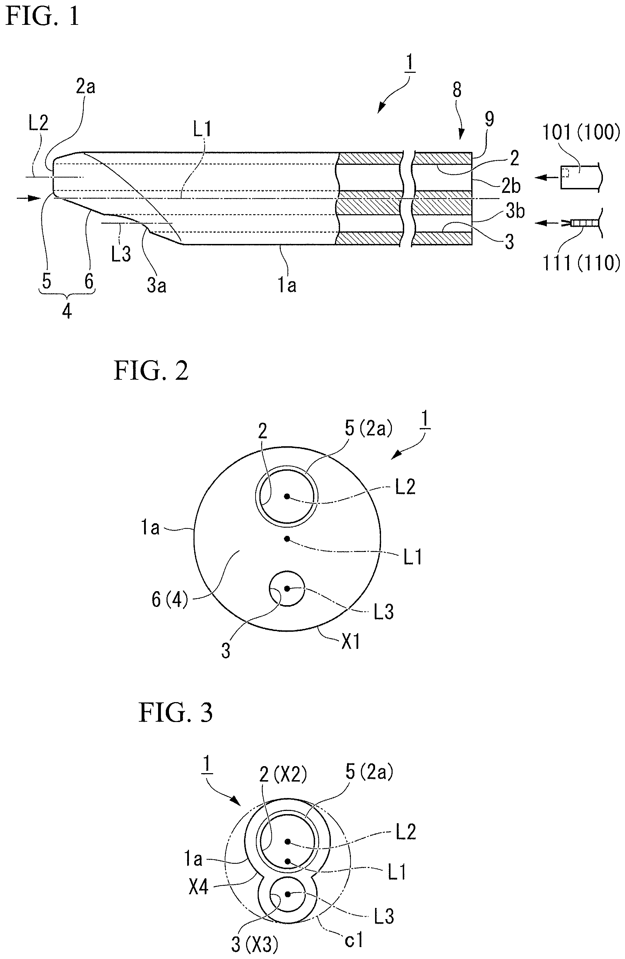

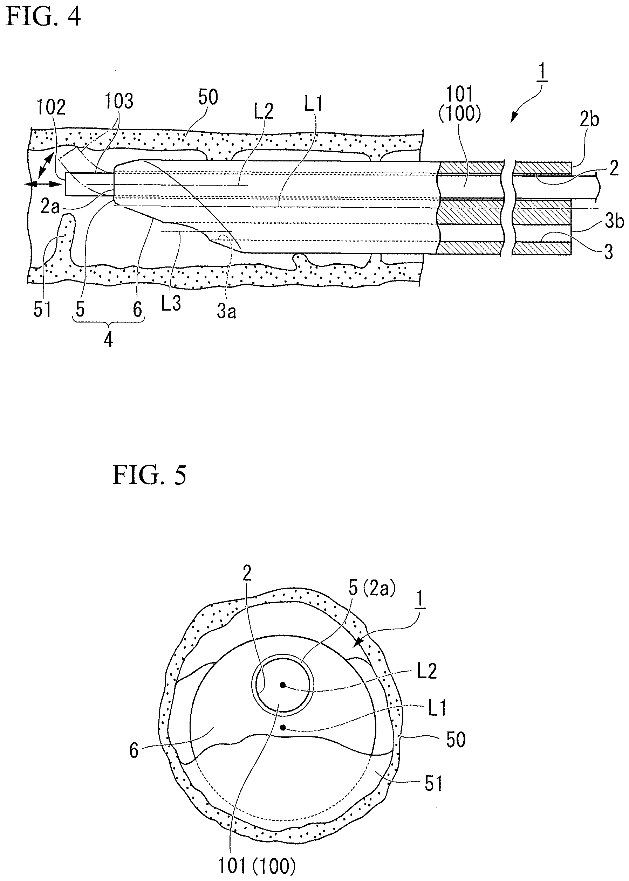

A first embodiment of the invention will be described. FIG. 1 is a side view showing a portion of an overtube according to the present embodiment in a section. FIG. 2 is a front view of the overtube according to the present embodiment. FIG. 3 is a front view showing another configuration example of the overtube according to the present embodiment. FIGS. 4 to 9 are views for explaining the operation of the overtube according to the present embodiment.

An overtube 1 according to the present embodiment shown in FIG. 1 is a medical instrument that guides an endoscope 100 and a treatment tool 110 to a treatment target site in the body.

As shown in FIG. 1, the overtube 1 is an elongated tubular member that has a first lumen 2 and a second lumen 3 formed therein and is formed to extend in a direction of a predetermined central axis L1. The overtube 1 has a tube part having the first lumen 2 and the second lumen 3 formed therein, and a distal end part 4 to be described below. As shown in FIG. 2, the overtube 1 has a circular sectional shape orthogonal to a longitudinal direction. The predetermined central axis L1 of the overtube 1 passes through the center of a circular profile X1 in a section orthogonal to the longitudinal direction of the overtube 1. The sectional shape orthogonal to the longitudinal direction of the overtube 1 may not be necessarily circular, according to the configuration of the first lumen 2 and the second lumen 3. For example, as shown in FIG. 3, an outside profile shape X4 of the overtube 1 in the section orthogonal to the longitudinal direction of the overtube 1 may follow a profile X2 of the first lumen 2 and a profile X3 of the second lumen 3. In this case, the central axis L1 of the overtube 1 refers to an axis passing through the center of a minimum circle C1 surrounding both the first lumen 2 and the second lumen 3. In a case where the section of the overtube 1 is not circular, the central axis L1 of the overtube 1 may be simply the longitudinal axis of the overtube 1.

The first lumen 2 shown in FIG. 1 formed from a through-hole that opens at a distal end surface 5 and a proximal end surface 9 of the overtube 1. The dimension of the first lumen 2 is set to correspond to the configuration of an insertion part 101 of the endoscope 100 attached to the overtube 1. An inner diameter of the first lumen 2 is slightly larger than the outer diameter of the insertion part 101 of the endoscope 100 used by being attached to the overtube 1, and has a dimension such that the insertion part 101 of the endoscope 100 may be rotated within the first lumen 2 with a centerline L2 of the first lumen 2 as a rotation center. The centerline L2 of the first lumen 2 runs substantially along the predetermined central axis L1 in the overtube 1.

The second lumen 3 formed from a through-hole that opens at an outer surface of the distal end part 4 of the overtube 1 and the proximal end surface 9 of the overtube 1. The dimension of the second lumen 3 is set to correspond to the configuration of an insertion part 111 of the treatment tool 110 attached to the overtube 1. An inner diameter of the second lumen 3 is slightly larger than the outer diameter of the insertion part 111 of the treatment tool 110 used by being attached to the overtube 1, and has a dimension such that the insertion part 111 of the treatment tool 110 may be rotated within the second lumen 3 with a centerline L3 of the second lumen 3 as a rotation center. The centerline L3 of the second lumen 3 runs substantially along the predetermined central axis L1 in the overtube 1.

In the present embodiment, although the number of second lumens 3 is one, the number of second lumens 3 is not limited to one. The overtube 1 may be configured with a plurality of the second lumens 3 that corresponds to the number of treatment tools 110 to be used in a procedure using the overtube 1 or is more than the number of treatment tools 110 to be used in a procedure using the overtube 1.

The distal end part 4 of the overtube 1 has the distal end surface 5 and a tapered surface 6. The distal end surface 5 of the overtube 1 includes a distal opening end 2a of the first lumen 2. The distal end surface 5 is a plate orthogonal to the central axis L1 of the overtube 1. As shown in Figs. 1 and 2, the distal end surface 5 of the overtube 1 in the present embodiment consists only of a profile portion of the distal opening end 2a of the first lumen 2 by forming the tapered surface 6 having a shape to be described below. The distal end surface 5 of the overtube 1 forms the distal opening end 2a of the first lumen 2 such that the center (centerline L2) of the distal opening end 2a of the first lumen 2 is located at a position apart from the central axis L1 of the overtube 1.

The tapered surface 6 is inclined with respect to the central axis L1 of the overtube 1 such that the tapered surface 6 gradually approaches the first lumen 2 as it moves from a proximal side (proximal end surface 9 side) to a distal side (distal end surface 5 side) in the direction of the central axis L1 of the overtube 1. The tapered surface 6 of the present embodiment is curved to form a portion of a conical surface such that the diameter thereof is gradually reduced toward the distal opening end 2a of the first lumen 2 from an outer peripheral surface 1a of the overtube 1. The tapered surface 6 of the present embodiment forms, for example, a portion of a conical surface in a cone that widens toward the proximal side of the overtube 1 about the centerline L2 of the first lumen 2. The tapered surface 6 of the overtube 1 forms a distal opening end 3a of the second lumen 3. The distal opening end 3a of the second lumen 3 is spaced apart from the distal opening end 2a of the first lumen 2.

The inclination angle of the tapered surface 6 with respect to the central axis L1 of the overtube 1 is not particularly limited if the inclination angle is less than 90 degrees. In the present embodiment, the tapered surface 6 consists only of the conical surface, but this configuration is not indispensable.

A proximal end part 8 of the overtube 1 is a part disposed outside the body in use of the overtube 1. The proximal end part 8 of the overtube 1 has the proximal end surface 9 that forms a proximal opening end 2b of the first lumen 2 and a proximal opening end 3b of the second lumen 3.

The operation of the overtube 1 according to the present embodiment will be described.

The overtube 1 according to the present embodiment is used in order to guide the endoscope 100 and the treatment tool 110 to the treatment target site in the body (including the inside of a gastrointestinal tract 50 shown in FIG. 4), in a case where treatment is performed on the treatment target site, using the endoscope 100 and the treatment tool 110. In the following, the operation of the overtube 1 according to the present embodiment will be shown by showing a process in which the overtube 1 according to the present embodiment is inserted into the gastrointestinal tract 50.

In a case where the overtube 1 is inserted into the gastrointestinal tract 50, first, the endoscope 100 is attached to the overtube 1 in order to ascertain the position of the overtube 1 within the gastrointestinal tract 50 and a state within the gastrointestinal tract 50 (refer to FIG. 4). Although the configuration of the endoscope 100 is not particularly limited, in the present embodiment, a so-called direct view type flexible endoscope 100 is attached to the overtube 1.

As shown in FIG. 4, in a process in which the endoscope 100 is attached to the overtube 1, firstly a distal end side of the insertion part 101 of the endoscope 100 is inserted into the first lumen 2 from the proximal opening end 2b of the first lumen 2 of the overtube 1. The insertion part 101 of the endoscope 100 is inserted toward the distal opening end 2a from the proximal opening end 2b of the first lumen 2. The insertion of the endoscope 100 into the first lumen 2 is stopped in a place where a distal end of the insertion part 101 of the endoscope 100 protrudes from the distal opening end 2a of the first lumen 2. The amount of protrusion of the endoscope 100 from the distal opening end 2a of the first lumen 2 may be appropriately set to a value such that the inside of the gastrointestinal tract 50 may be suitably viewed by the endoscope 100. For example, in order to move an imaging unit 102 at a distal end of the endoscope 100, the endoscope 100 may protrude from the distal opening end 2a of the first lumen 2 with a protrusion amount such that some or all of the active curved part 103 disposed on the proximal side of the imaging unit 102 is exposed from the distal opening end 2a of the first lumen 2.

The treatment tool 110 may not be inserted through the second lumen 3 until the distal end part 4 of the overtube 1 is guided to the vicinity of the treatment target site. In a case where the treatment tool 110 is inserted through the second lumen 3 in the process until the distal end part 4 of the overtube 1 is guided to the vicinity of the treatment target site, it is preferable that all a distal portion of the insertion part 111 of the treatment tool 110 is located within the second lumen 3. If the distal portion of the insertion part 111 of the treatment tool 110 is within the second lumen 3, a distal portion of the treatment tool 110 is protected so as not to contact an inner wall of the gastrointestinal tract 50. Simultaneously, if the distal portion of the insertion part 111 of the treatment tool 110 is within the second lumen 3, the inner wall of the gastrointestinal tract 50 is protected so as not to contact the distal portion of the treatment tool 110.

For example, in a case where the overtube 1 is inserted into the gastrointestinal tract 50 having a fold 51 as shown in FIG. 4, such as an intestinal tract, insertion resistance may be generated in the overtube 1 when the distal end part 4 of the overtube 1 is pushed back by the fold 51 of the gastrointestinal tract 50. Even in a case where the overtube 1 is inserted into a portion where the gastrointestinal tract 50 has a small diameter due to stenosis, peristalsis, or the like of the gastrointestinal tract 50, the insertion resistance may be similarly generated.

In the present embodiment, as shown in FIG. 4, the distal end of the insertion part 101 of the endoscope 100 protrudes from the distal opening end 2a of the first lumen 2 of the overtube 1, and the endoscope 100 is located more distal than the overtube 1 in a state where the endoscope 100 is attached to the overtube 1. Since the overtube 1 has the second lumen 3 for attaching the treatment tool 110 and the first lumen 2 for attaching the endoscope 100, the overtube has a larger diameter than the insertion part 101 of the endoscope 100 as a whole. In the present embodiment, in the distal end part 4 of the overtube 1, the outer diameter in the vicinity of the distal opening end 2a of the first lumen 2 is slightly larger than the outer diameter of the insertion part 101 of the endoscope 100.

In a case where the overtube 1 is further moved within the gastrointestinal tract 50 so as to ride over the fold 51, the distal end of the endoscope 100 moves to ride over the fold 51 earlier than the distal end of the overtube 1. As shown in Figs. 4 and 5, since the insertion part 101 of the endoscope 100 has a smaller diameter than that of the overtube 1, the insertion resistance is not easily generated in the endoscope 100 in a state where the overtube 1 is located in front of the fold 51 serving as a ride-over target within the gastrointestinal tract 50. When the endoscope 100 is moved so as to ride over the fold 51 under observation within the gastrointestinal tract 50 using the endoscope 100, the fold 51 can also be avoided by curving the active curved part 103 of the endoscope 100.

In a case where the insertion part 101 of the endoscope 100 rides over the fold 51, the overtube 1 and the endoscope 100 may be integrally moved in the direction of the central axis L1 of the overtube 1, or the overtube 1 may not be moved but the insertion part 101 of the endoscope 100 may be moved toward the distal side of the overtube 1 with respect to the overtube 1.

After the insertion part 101 of the endoscope 100 rides over the fold 51, subsequently, the overtube 1 is moved toward the distal side such that the overtube 1 rides over the fold 51. In the distal end part 4 of the overtube 1, the vicinity of the distal opening end 2a of the first lumen 2 firstly contacts the fold 51 and pushes the fold 51. Since the fold 51 is in contact with the tapered surface 6 in the distal end part 4 of the overtube 1, if the overtube 1 is moved toward the distal side in the direction of the central axis L1 of the overtube 1, the tapered surface 6 moves the fold 51 toward an insertion direction side of the overtube 1 to the gastrointestinal tract 50 and a radial outer side of the gastrointestinal tract 50. That is, the fold 51 of the gastrointestinal tract 50 is stretched and extended by the tapered surface 6 in a process in which the overtube 1 is pushed to the distal side.

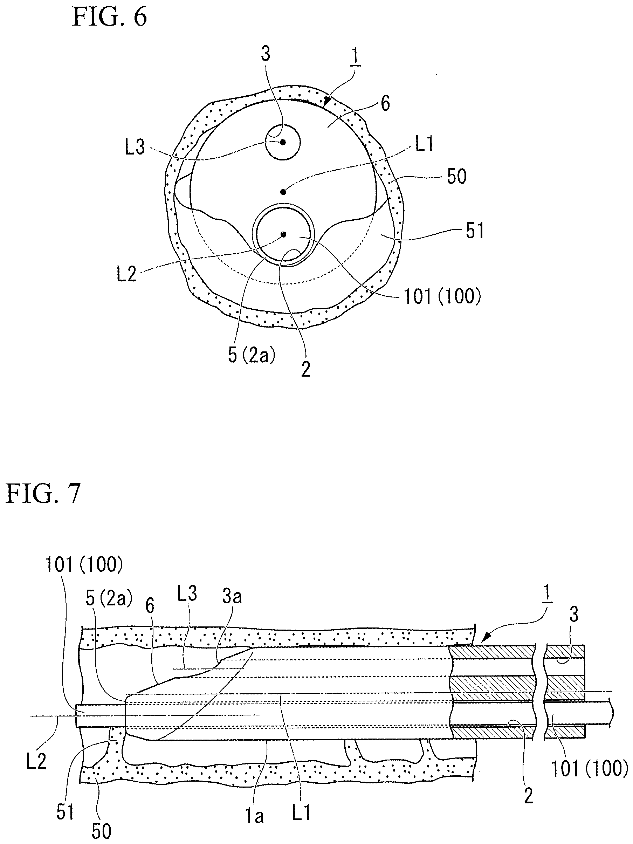

By the operation of inserting the overtube 1, the tapered surface 6 stretches and extends the fold 51 of the gastrointestinal tract 50 to such a degree that the overtube 1 is inserted. In the present embodiment, although a force with that the fold 51 pushes back the tapered surface 6 is smaller than the insertion resistance in a case where the tapered surface 6 is not formed, the insertion resistance is generated in the overtube 1 due to the force. In order to make this insertion resistance smaller, the operator can rotate the overtube 1 about the insertion part 101 of the endoscope 100, as shown in Figs. 5 and 6, in a state where the tapered surface 6 is in contact with the fold 51. Instead of rotating the overtube 1 about the insertion part 101 of the endoscope 100, the overtube 1 and the endoscope 100 may be rotated with the central axis L1 of the overtube 1 as a rotation center. Through these kinds of rotation, the distal end part 4 of the overtube 1 rotates within the gastrointestinal tract 50 about the insertion part 101 of the endoscope 100.

The major advantages obtained by rotating the overtube 1 within the gastrointestinal tract 50 are shown as the follows.

As a first advantage, when the distal end part 4 of the overtube 1 rotates as the center of the insertion part 101 of the endoscope 100, as shown in Figs. 6 and 7, a positional relationship in which the overtube 1 becomes the furthest from the fold 51 may be obtained. The fold 51 within the gastrointestinal tract 50 is not always limited to extending over the entire circumference of the gastrointestinal tract 50, and may be a site where the fold 51 protrudes high within the gastrointestinal tract 50, and a site where the fold 51 is low. If the overtube 1 is rotated in a state where the insertion part 101 of the endoscope 100 first rides over the fold 51, the insertion part 101 of the endoscope 100 is supported by the fold 51, and thereby the insertion part 101 of the endoscope 100 serves as a fulcrum. As a result, the overtube 1 moves to the insertion part 101 of the endoscope 100. That is, the overtube 1 may be rotated within the gastrointestinal tract 50 to avoid the fold 51 such that the overtube 1 performs an eccentric motion with the insertion part 101 of the endoscope 100 as a fulcrum. Accordingly, the insertion resistance generated in the overtube 1 becomes small.

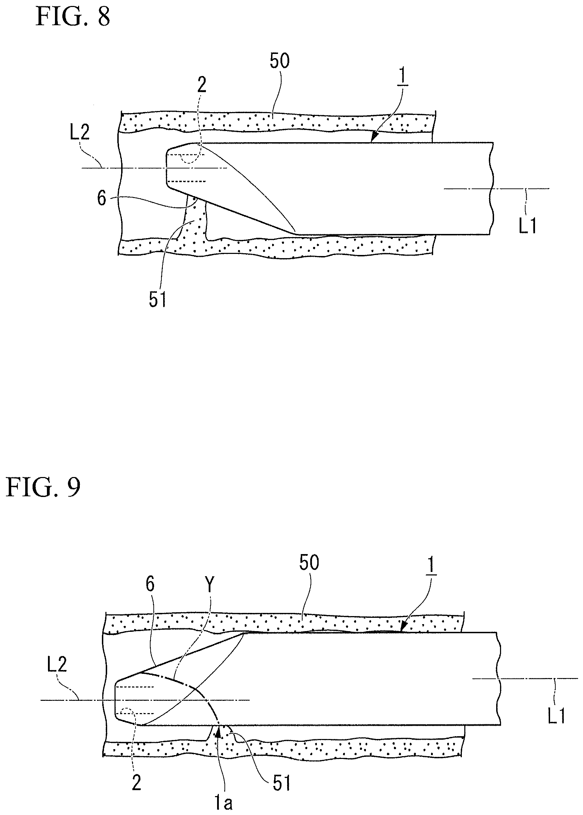

As a second advantage, when the distal end part 4 of the overtube 1 is further inserted rotating about the insertion part 101 of the endoscope 100, as shown in Figs. 8 and 9, the fold 51 relatively moves along a spiral path Y in the tapered surface 6, and the fold 51 is stretched and extended. The spiral path Y in the tapered surface 6 is a path that forms a portion of a spiral centered on the centerline L2 of the first lumen 2 in the tapered surface 6. The spiral path Y in the tapered surface 6 is longer than a straight line connecting a distal end and a proximal end in the tapered surface 6 together. For this reason, by stretching and extending the fold 51 along the path Y that forms a portion of the spiral centered on the centerline L2 of the first lumen 2 in the tapered surface 6, the fold 51 is moved along an inclined surface of an angle gentler than an angle at which the tapered surface 6 is formed with respect to the central axis L1 of the overtube 1. Accordingly, the insertion resistance generated in the overtube 1 becomes small.

As described above, the overtube 1 according to the present embodiment can reduce the insertion resistance in use of the overtube 1 by providing the tapered surface 6 as compared to a case where the tapered surface 6 is not provided.

The centerline L2 of the first lumen 2 of the overtube 1 according to the present embodiment is at the position apart from the predetermined central axis L1 of the overtube 1. For this reason, if the overtube 1 is rotated in a state where the insertion part 101 of the endoscope 100 disposed within the first lumen 2 is inserted into a region where the inner diameter of the gastrointestinal tract 50, such as the fold 51 of the gastrointestinal tract 50, becomes small, the overtube 1 performs the eccentric motion with the insertion part 101 of the endoscope 100 as a fulcrum. As a result, the overtube 1 may be rotated within the gastrointestinal tract 50 to avoid the fold 51 or the like of the gastrointestinal tract 50.

Since the centerline L2 of the first lumen 2 is at the position apart from the predetermined central axis L1 of the overtube 1, the first lumen 2 and the second lumen 3 may be arranged with high density in a section to the longitudinal direction of the overtube 1. For this reason, the overtube 1 may be made to have a small diameter.

### (Modification Example 1)

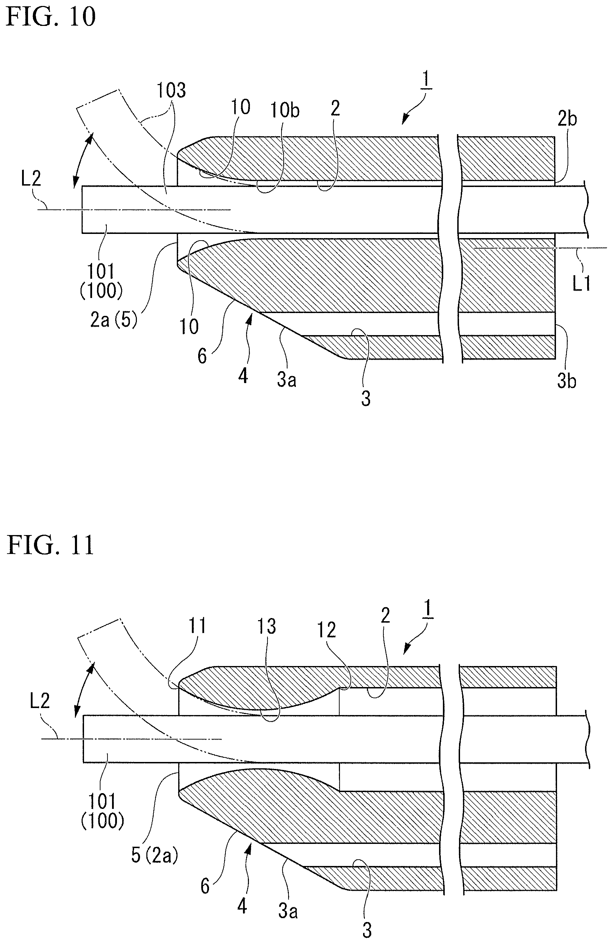

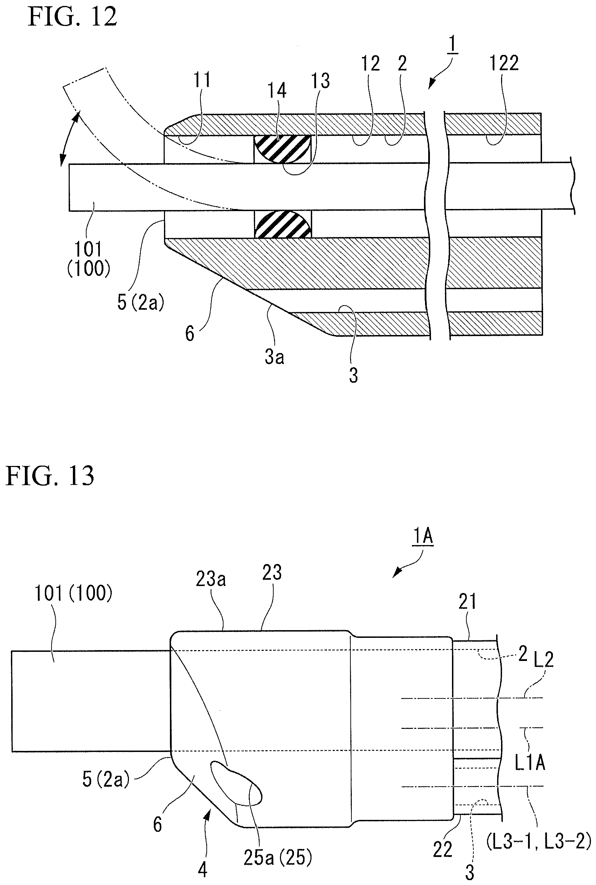

Next, Modification Example 1 of the present embodiment will be described. FIG. 10 is a sectional view showing a configuration example of the present modification example. FIG. 11 is a sectional view showing another configuration example in the present modification example. FIG. 12 is a sectional view showing still another configuration example in the present modification example.

As shown in FIG. 10, the present modification example is different from the first embodiment in terms of the inner surface shape of the first lumen 2 in the distal end part 4 of the overtube 1.

The first lumen 2 in the present modification example, as shown in FIG. 10, has a curved surface 10 having a gradually smaller diameter until an inner diameter having a slight clearances with respect to the outer diameter of the insertion part 101 of the endoscope 100 is obtained, as it moves from the distal opening end 2a of the first lumen 2 to the proximal side. Since the curved surface 10 is formed at a proximal portion of the distal opening end 2a of the first lumen 2, the curved surface 10 supports the insertion part 101 of the endoscope 100 such that the centerline of the insertion part 101 of the endoscope 100 and the centerline L2 of the first lumen 2 substantially coincide with each other. The curved surface 10 forms a space required in order for the active curved part 103 provided in the insertion part 101 of the endoscope 100 to operate, in the vicinity of the distal opening end 2a of the first lumen 2.

In the present embodiment, the distal opening end 2a of the first lumen 2 is larger than the inner diameter of the first lumen 2 ranging from a proximal end 10b of the curved surface 10 to the proximal opening end 2b of the first lumen 2. In the first lumen 2, the centerline of the insertion part 101 of the endoscope 100 and the centerline L2 of the first lumen 2 substantially coincide with each other in a region ranging from the proximal end 10b of the curved surface 10 to the proximal opening end 2b of the first lumen 2. For this reason, meandering of the insertion part 101 of the endoscope 100 within the first lumen 2 does not easily occur. As a result, when the overtube 1 is rotated about the insertion part 101 of the endoscope 100 or the insertion part 101 of the endoscope 100 is rotated with respect to the overtube 1, a rotating force applied by the operator on the proximal side is suitably transmitted to the distal side.

The curved shape of the curved surface 10 may be such a shape that the active curved part 103 runs along the curved surface 10, when the active curved part 103 provided in the insertion part 101 of the endoscope 100 is brought into a curved state with a largest curvature. If the active curved part 103 has a shape that runs along the curved surface 10 when the active curved part 103 is brought into the curved state with the largest curvature, the curving performance of the active curved part 103 of the endoscope 100 is not limited by the overtube 1, and the movable range of the endoscope 100 may be widely maintained.

A configuration in which the space required in order for the active curved part 103 provided in the insertion part 101 of the endoscope 100 to operate may be secured in the vicinity of the distal opening end 2a of the first lumen 2 is not limited to the curved surface 10.

For example, the overtube 1, as shown in FIG. 11, may have larger-diameter parts 11 and 12 and a small-diameter part 13 within a predetermined region in the vicinity of the distal end part 4 of the overtube 1. The larger-diameter parts 11 and 12 have a relatively large inner diameter on the distal side and on the proximal side in the direction of the centerline L2 of the first lumen 2. The small-diameter part 13 is located between the respective larger-diameter parts 11 and 12. The small-diameter part 13 holds the insertion part 101 of the endoscope 100 in a forward and backward movable manner such that the centerline of the insertion part 101 of the endoscope 100 and the centerline L2 of the first lumen 2 substantially coincide with each other.

In an example shown in FIG. 12, the above-mentioned respective larger-diameter parts 11 and 12 are configured by having a larger inner diameter than the outer diameter of the insertion part 101 of the endoscope 100 in the whole region of the first lumen 2. Moreover, a ring part 14 that exhibits the same function as the small-diameter part 13 is provided between the larger-diameter parts 11 and 12. The ring part 14 is in contact with an outer peripheral surface of the insertion part 101 of the endoscope 100 (or approaches with a slight gap). The ring part 14 may be a member that maintains airtightness or watertight between the first lumen 2 and the insertion part 101.

### (Second Embodiment)

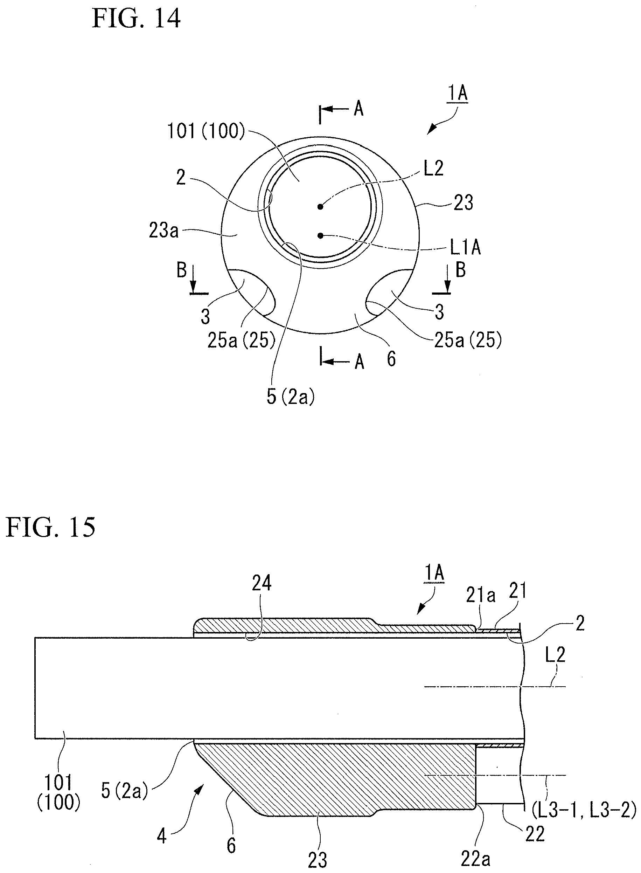

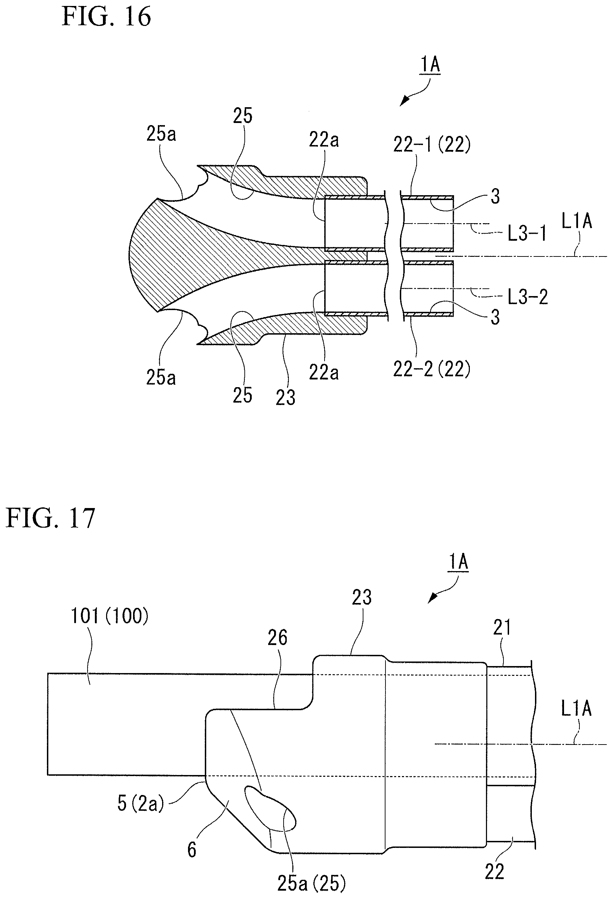

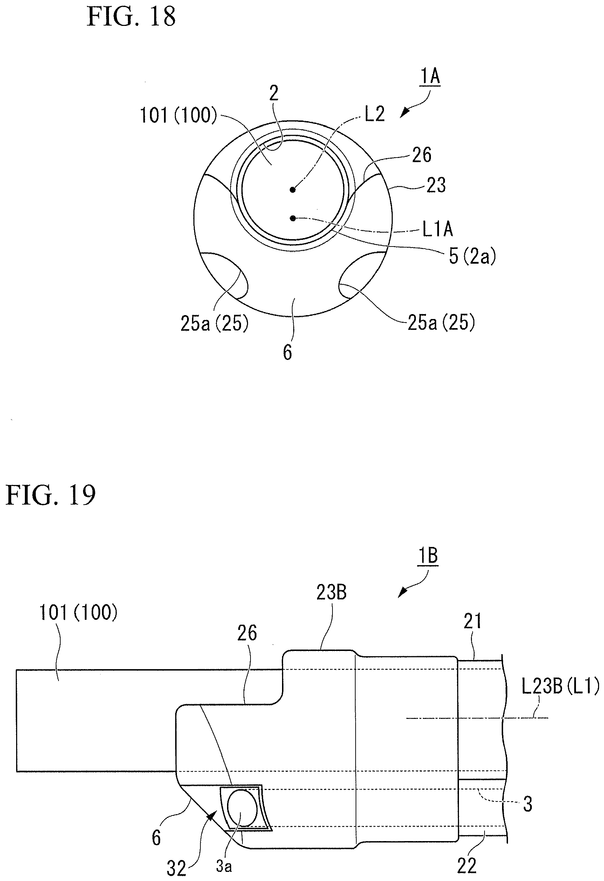

A second embodiment of the invention will be described. In the following respective embodiments, the same configuration elements as those of the above-mentioned first embodiment will be designated by the same reference signs as those of the first embodiment, and duplicate description thereof will be omitted. FIG. 13 is a side view showing a portion of an overtube according to the present embodiment. FIG. 14 is a front view of the overtube according to the present embodiment. FIG. 15 is a sectional view in line A-A of FIG. 14. FIG. 16 is a sectional view in line B-B of FIG. 14. FIG. 17 is a side view showing another configuration example of the overtube according to the present embodiment. FIG. 18 is a front view showing the other configuration example of the overtube according to the present embodiment.

An overtube 1A according to the present embodiment shown in FIG. 13 includes a first tube member 21, a second tube member 22, and a coupling member 23. The first tube member 21 is corresponding to the first lumen 2 disclosed in the above-mentioned first embodiment, and is configured to form a lumen. The second tube member 22 is corresponding to the second lumen 3 disclosed in the above-mentioned first embodiment, and is configured to form a lumen. The coupling member 23 is fixed to a distal portion of the first tube member 21 and a distal portion of the second tube member 22.

The overtube 1A has two second tube members 22 (reference signs 22-1 and 22-2 shown in FIG. 16) in order to be enable two treatment tools (not shown) to be simultaneously attached to the overtube 1A.

As shown in Figs. 15 and 16, the first tube member 21 and the second tube members 22 are provided such that centerlines (the centerline L2 of the first lumen 2 and centerlines L3-1 and L3-2 of the respective second lumens 3) thereof are parallel to each other. The distal end 21a of the first tube member 21 and the distal ends 22a of the second tube members 22 are held by the coupling member 23 in a state where the centerline L2 of the first tube member 21 and the centerlines L3-1 and L3-2 of the second tube members 22 are parallel to each other.

As shown in FIGS. 13 to 16, the coupling member 23 is corresponding to a substantially tubular member in which the distal end surface 5 and the tapered surface 6 disclosed in the first embodiment are formed. A first through-hole 24 (refer to FIG. 15) and second through-holes 25 (refer to FIG. 16) are formed inside the coupling member 23. The first through-hole 24 is corresponding to a portion of the first lumen 2 that communicates with the inside of the first tube member 21 and is disclosed in the above-mentioned first embodiment. The second through-holes 25 are corresponding to portions of the second lumens 3 that communicate with the insides of the second tube members 22 and are disclosed in the above-mentioned first embodiment.

The first through-hole 24 is a through-hole having a straight line serving as the centerline L2 of the first lumen 2 as a centerline. The inner diameter of the first through-hole 24 is larger than the outer diameter of the insertion part 101 of the endoscope 100 by such a clearance that the insertion part is capable of being advanced and retracted.

The second through-holes 25 are a pair of through-holes that has curved centerline serving as the centerlines L3-1 and L3-2 of the two second lumens 3. An opening end 25a of each second through-hole 25 on the distal side is corresponding to the distal opening end 3a of the second lumen 3 disclosed in the first embodiment. The opening end 25a of each second through-hole 25 on the distal side is configured within a region over a portion of the tapered surface 6 (to be described below) in the coupling member 23 and a portion of an outer peripheral surface 23a of the coupling member 23. The respective second through-holes 25 of the present embodiment, as the plan view shown in FIG. 16, are curved in mutually opposite directions so as to be axisymmetrical with the central axis L1 of the overtube 1A interposed therebetween. Each second through-hole 25 specifies a protruding direction of a treatment tool to be inserted into the inside of each second through-hole 25. The curved shape of each second through-hole 25 is not limited to the curved shape of the shown example, and may be a suitable shape, on the basis of the configuration of a procedure using the overtube 1A, a treatment tool to be attached to the overtube 1A, or the like.

As shown in Figs. 17 and 18, a cutout part 26 that reaches a portion of a distal portion of the first through-hole 24 may be formed in a distal portion in the coupling member 23. The cutout part 26 formed in the coupling member 23 maintains the movable range of the endoscope 100 widely, similar to the configuration of the first lumen 2 disclosed as the modification example of the above-mentioned first embodiment.

The tapered surface 6 formed in the coupling member 23 shown in FIG. 15 forms a portion of a conical surface having a centerline (that is, in the present embodiment, the centerline L2 of the first lumen 2) of the first through-hole 24 as an axis.

A predetermined central axis L1A in the overtube 1A is the center of a circular profile that surrounds the first tube member 21 and the second tube members 22 in the section orthogonal to the centerlines L2, L3-1, and L3-2 of the first tube member 21 and the second tube members 22, and coincides with a centerline L23 of the coupling member 23.

The overtube 1A can reduce the insertion resistance in use of the overtube 1A as compared to a case where the tapered surface 6 is not provided, similar to the overtube 1 of the above-mentioned first embodiment.

### (Third Embodiment)

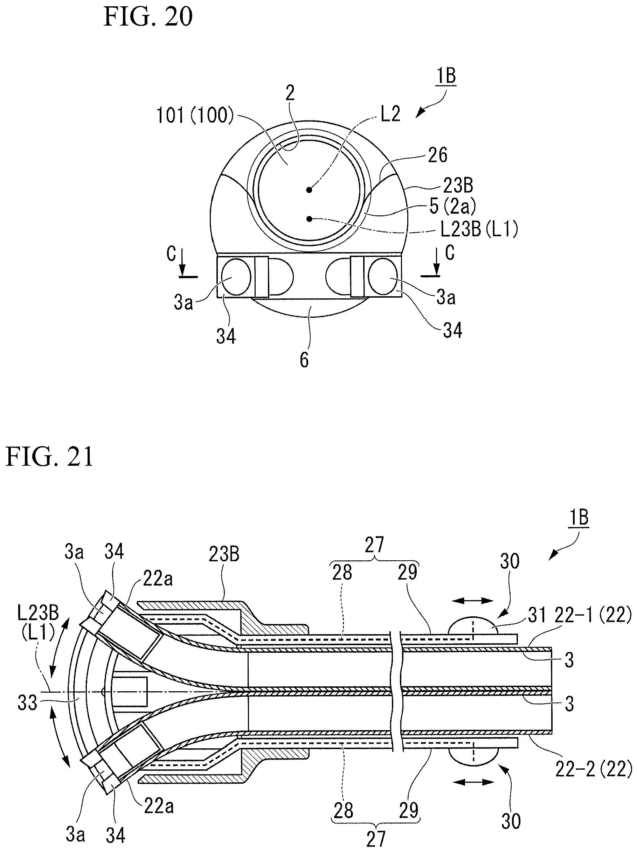

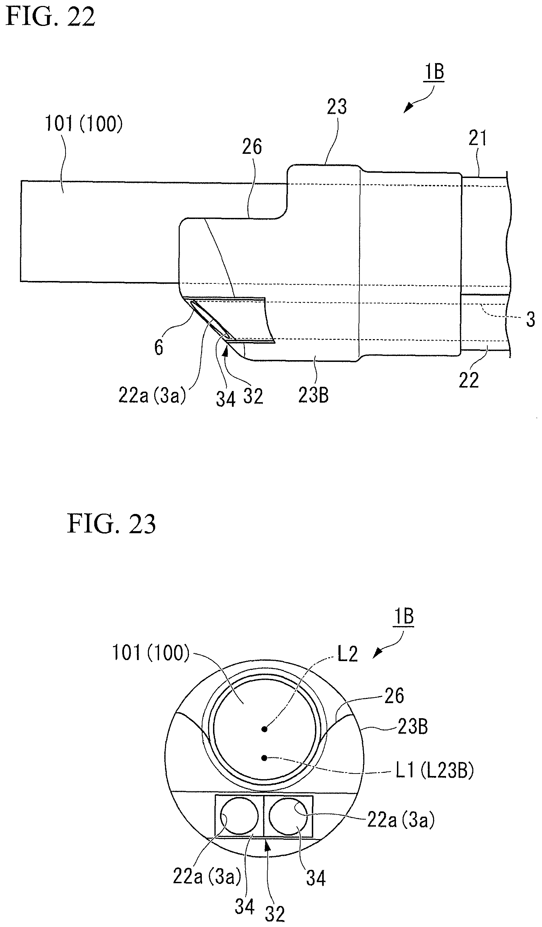

A third embodiment of the invention will be described. FIG. 19 is a side view showing a portion of an overtube according to the present embodiment. FIG. 20 is a front view of the overtube according to the present embodiment. FIG. 21 is a cross-sectional view in line C-C of FIG. 20. FIG. 22 is a view for explaining the operation of the overtube according to the present embodiment. FIG. 23 is a view for explaining the operation of the overtube according to the present embodiment.

As shown in Figs. 19 and 20, the configuration of an overtube 1B according to the present embodiment is different from that of the above-mentioned first embodiment and second embodiment in that the overtube 1B has a movable mechanism 32 that can move the positions of the distal opening ends 3a of the two second lumens 3, respectively.

The overtube 1B includes a coupling member 23B having the movable mechanism 32, instead of the coupling member 23 disclosed in the above-mentioned second embodiment. Moreover, the overtube 1B, as shown in FIG. 21, includes power transmission parts 27 and operating parts 30. Each power transmission part 27 extends parallel to the first tube member 21 and each second tube member 22, and has an operating wire 28 coupled to the movable mechanism 32. Each operating part 30 is provided in order to operate to move the operating wire 28 forward and backward in the vicinity of the proximal ends of the first tube member 21 and the second tube member 22.

As shown in FIG. 20, the coupling member 23B of the overtube 1B has a centerline L23B, substantially similar to the coupling member 23 disclosed in the second embodiment. The centerline L23B of the coupling member 23B of the present embodiment coincides with the central axis L1 of the overtube 1B.

As shown in FIG. 21, the movable mechanism 32 has a guide member 33 and tube holding members 34. The guide member 33 is an elongated member that is arranged in a direction different from a direction in which the central axis L1 of the overtube 1B extends. Each tube holding member 34 is assembled to the guide member 33 so as to be advanced and retracted along the guide member 33.

The guide member 33 extends in a curved manner so as to form, for example, a portion of a circular arc in the plan view shown in FIG. 21. The shape of the guide member 33 specifies the position and orientation of an opening of the distal end 22a of each second tube member 22 within a predetermined movement range. The guide member 33 may extend in a curved manner, or may extend linearly. The present embodiment, one guide member 33 may be used for a double purpose in order to specify the movement ranges of the openings of the distal ends 22a of the plurality of second tube members 22.

The tube holding member 34 is fixed to the second tube member 22 in an outer peripheral portion of the opening of the distal end 22a of the second tube member 22. In the present embodiment, instead of the second through-holes 25 formed in the coupling member 23 in the above-mentioned second embodiment, the second tube members 22 themselves are deformed in a curved manner. According to the position of the tube holding member 34 with respect to the guide member 33, each second tube member 22 can also be brought into a straight tube shape that extends parallel to the centerline L23B of the coupling member 23B inside the coupling member 23B.

In the present embodiment, as shown in FIGS. 20, 21, and 23, the tube holding member 34 is movable along the guide member 33 so as to correspond to the operation of the operating wire 28 in each operating part 30 shown in FIG. 21. In a state (refer to Figs. 22 and 23) where the second tube member 22 is brought into the straight tube shape in inside the coupling member 23B, the tube holding member 34 is flush with the tapered surface 6 of the coupling member 23B or is at a position where the tube holding member recedes into the inside of the coupling member 23B further than the tapered surface 6. For this reason, the tube holding member 34 and the second tube member 22 do not easily contact tissue, such as the gastrointestinal tract 50 (for example, refer to FIG. 4) when the second tube member 22 is brought into the straight tube shape inside the coupling member 23B. For example, the tube holding member 34 and the second tube member 22 are not easily caught in a projection-like portion, such as the fold 51 of the gastrointestinal tract 50, when the second tube member 22 is brought into the straight tube shape inside the coupling member 23B.

As shown in FIG. 21, the power transmission part 27 has the operating wire 28 and the sheath 29 through which the operating wire 28 is inserted in a forward and backward movable manner. In the present embodiment, one power transmission part 27 is provided for each of the two second tube members 22 that form the two second lumens 3.

That is, the overtube 1B according to the present embodiment has the two power transmission parts 27. A total of two operating wires 28 provided for the two power transmission parts 27, respectively, are independently operated to be advanced and retracted by the operating parts 30.

A distal portion of each operating wire 28 is directed so as to become parallel to a direction in which the guide member 33 extends, and is attached to the coupling member 23B. A distal end of each operating wire 28 is coupled to the tube holding member 34. For this reason, the forward and backward movement operation of each operating wire 28 in the direction of the centerline of each operating wire 28 is transmitted to each tube holding member 34 as the forward and backward operation of the tube holding member 34 in the direction in that the guide member 33 extends.

The operating part 30 is disposed in the vicinity of proximal ends of the first tube member 21 and the second tube member 22 in order to move a proximal end of each of the two operating wires 28 in the direction of the centerline of each operating wire 28. For example, the operating part 30 has a slider 31 coupled to the proximal end of each operating wire 28.

The operation of the overtube 1B according to the present embodiment will be described.

Since it is not necessary to use the treatment tool 110 in the process in which the overtube 1B is guided up to a treatment target site through the inside of the gastrointestinal tract 50, the second tube member 22 is brought into a straight tube shape (states shown in Figs. 22 and 23) inside the coupling member 23B. In this case, since the opening of the distal end 22a of the second tube member 22 is flush with the tapered surface 6 of the coupling member 23B or is located closer to an inner side than the tapered surface 6, a possibility that the second tube member 22 may contact the fold 51 or the like that touches the tapered surface 6 and cause insertion resistance is suppressed to be low.

When the operator operates the operating part 30 as necessary after the overtube 1B is guided to the treatment target site, the operating wire 28 can move the tube holding member 34 along the guide member 33, and as shown in FIGS. 19 to 21, the distal portion of the second tube member 22 may be curved. That is, the overtube 1B can move the distal opening end 3a of the second lumen 3 with respect to the distal opening end 2a of the first lumen 2 according to the forward and backward movement operation to the operating wire 28 in the operating part 30. Accordingly, the position and orientation in which the treatment tool 110 protrudes from the opening of the second tube member 22 on the distal side may be adjusted.

Also in the present embodiment, the insertion resistance may be reduced similar to the first embodiment by rotating the overtube 1 B, similar to the first embodiment, within the gastrointestinal tract 50.

### (Fourth Embodiment)

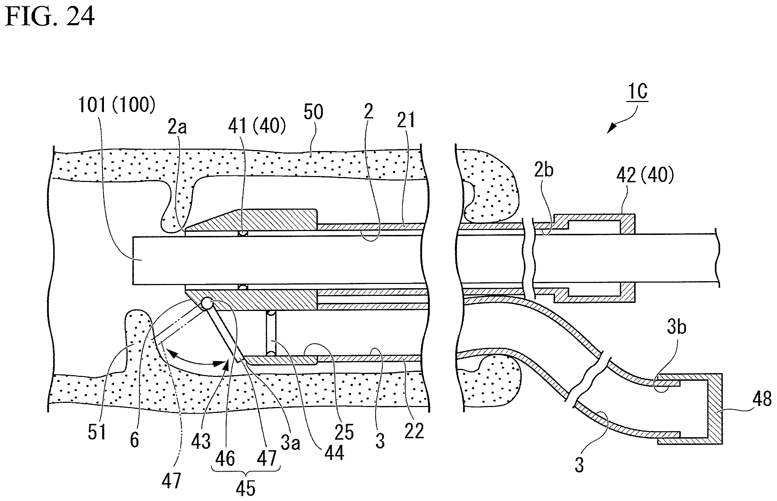

A fourth embodiment of the invention will be described. FIG. 24 is a sectional view showing an overtube according to the present embodiment.

The configuration of an overtube 1C according to the present embodiment shown in FIG. 24 is different from the overtube 1A (refer to FIG. 13) according to the above-mentioned second embodiment in that the overtube 1C further has a first sealing part 40 and a second sealing part 43. The first sealing part 40 airtightly seals between the first lumen 2 and an endoscope 100. The second sealing part 43 airtightly seals between the second lumen 3 and the treatment tool 110 (for example, refer to FIG. 1).

The first sealing part 40 of the present embodiment has a configuration in which it is assumed that the endoscope 100 is always inserted into the first lumen 2 in use of the overtube 1C. That is, the first sealing part 40 of the present embodiment is a first annular member 41 attached to an inner surface of the first lumen 2 so as to fill a gap between the inner surface of the first lumen 2 and an outer surface of the insertion part 101 of the endoscope 100.

The first sealing part 40 may have a valve or a cap 42 for sealing the first lumen 2 in a state where the insertion part 101 of the endoscope 100 is not inserted into the first lumen 2 of the overtube 1C at the proximal opening end 2b of the first lumen 2, instead of the first annular member 41 or in addition to the first annular member 41.

The second sealing part 43 of the present embodiment has a second annular member 44 and a cover part 45. The second annular member 44 fills a gap between an inner surface of the second lumen 3 and an outer surface of a treatment tool in a state where the treatment tool is inserted into the second lumen 3. The cover part 45 closes the second lumen 3 in a state where the treatment tool is not inserted into the second lumen 3.

The cover part 45 has a connecting shaft member 46, a cover member 47, and a biasing member (not shown). The connecting shaft member 46 is fixed to the coupling member 23 of the overtube 1C. The cover member 47 is a plate-shaped member that is rotatably coupled to the coupling member 23 via the connecting shaft member 46 with a centerline of the connecting shaft member 46 as a rotation center. The biasing member generates a biasing force that causes the cover member 47 to abut against the distal opening end 3a of the second lumen 3.

The cover member 47 covers the second through-hole 25 and airtightly seals the distal opening end 3a of the second lumen 3 when the cover member 47 abuts an opening end (that is, the distal opening end 3a of the second lumen 3 in the present embodiment) of the second through-hole 25 on the distal side. The cover member 47 is rotated with the centerline of the connecting shaft member 46 as the rotation center so as to be spaced apart from the opening end of the second through-hole 25 of the distal side to the distal side when the cover member 47 is pushed toward the distal side from the proximal side in the direction of the centerline of the second through-hole 25. The cover member 47 is pushed and made rotatable as described above by a treatment tool inserted into the second through-hole 25. That is, in the present embodiment, when the treatment tool (not shown) inserted into the inside of the second through-hole 25 pushes the surface of the cover member 47 on the proximal side toward the distal side, the cover member 47 is opened by a pressing force.

The second sealing part 43 may have a valve or a cap 48 for sealing the second lumen 3 in a state where no treatment tool is inserted into the second lumen 3 of the overtube 1C, at the proximal opening end 3b of the second lumen 3, in addition the second annular member 44 and cover part 45.

In use of the overtube 1C according to the present embodiment, ambient air is sent into the gastrointestinal tract 50 in order to form a sufficient space for inserting the overtube 1C into the gastrointestinal tract 50. The sending of the air into the gastrointestinal tract 50 is performed by well-known air-sending means provided in the endoscope 100 to be inserted through the first lumen 2 of the overtube 1C. Since a gap between the first lumen 2 and the endoscope 100 is made airtight by the first sealing part 40 and the second lumen 3 is sealed by the cover member 47, the gastrointestinal tract 50 is swollen with the ambient air sent into the gastrointestinal tract 50 due to the sending of the air by the endoscope 100. Accordingly, a wide space where the overtube 1C is easily inserted is formed by stretching the fold 51 of the gastrointestinal tract 50, for example. Also in the present embodiment, the fold 51 may be stretched and extended using the tapered surface 6, similar to the above-mentioned first embodiment.

When the second lumen 3 of the overtube 1C is closed by the cover member 47, tissue, such as the fold 51, does not enter the inside of the second lumen 3. For this reason, since the tissue, such the fold 51, cannot be easily caught in the distal opening end 3a of the second lumen 3, the insertion resistance of the overtube 1C may be made lower as compared to a case where the cover member 47 is not provided.

When a treatment tool is introduced into the gastrointestinal tract 50 through the second lumen 3, the cover member 47 is moved to the distal side using the treatment tool inserted into the second lumen 3. Accordingly, the treatment tool can protrude into the gastrointestinal tract 50 so as to be used for treatment. If the treatment using the treatment tool is completed, the treatment tool may be pulled back into the second lumen 3, and if the treatment tool is pulled back into the second lumen 3, the cover member 47 is again closed due to the operation of the biasing member (not shown).

In this way, according to the overtube 1C according to the present embodiment, the gastrointestinal tract 50 may be swollen due to the sending of the air by the endoscope 100, or the like, the diameter of the gastrointestinal tract 50 may be increased, and the insertion resistance of the overtube 1C may be lowered. Also in the present embodiment, similar to the above-mentioned first embodiment, the overtube 1C may be rotated to stretch and extend the fold 51, or the overtube 1C may be moved so as to avoid the fold 51.

In the present embodiment, if the air-sending means is capable of sending air to such a degree that an atmospheric pressure difference between the inside of the gastrointestinal tract 50 and the second lumen 3 is caused, even if the cover part 45 does not have the biasing member, the cover member 47 may be brought into contact with the distal opening end 3a of the second lumen 3 due to the atmospheric pressure difference.

Although the embodiments of the invention have been described above in detail with reference to the drawings, specific configuration is not limited to these embodiments, and design changes are also included without departing from the scope of the invention. The invention is not to be considered as being limited by the foregoing description, and is limited only by the scope of the appended claims.

The configuration elements shown in the above-described respective embodiments and respective modification examples can be appropriately combined together.

### [Industrial Applicability]

According to the above-mentioned respective embodiments, the overtube with a small insertion resistance in use can be provided.

### [Reference Signs List]

- 1, 1A, 1B, 1C:: OVERTUBE
- 2:: FIRST LUMEN
- 2a:: DISTAL OPENING END
- 2b:: PROXIMAL OPENING END
- 3:: SECOND LUMEN
- 3a:: DISTAL OPENING END
- 3b:: PROXIMAL OPENING END
- 4:: DISTAL END PART
- 5:: DISTAL END SURFACE
- 6:: TAPERED SURFACE
- 8:: PROXIMAL END PART
- 9:: PROXIMAL END SURFACE
- 32:: MOVABLE MECHANISM
- 40:: FIRST SEALING PART
- 43:: SECOND SEALING PART
- 45:: COVER PART
- 47:: COVER MEMBER
- 100:: ENDOSCOPE
- 110:: TREATMENT TOOL

## Claims

1. An overtube that is capable of being inserted by an endoscope and a treatment tool and is elongated in a direction of a predetermined central axis, the overtube comprising:
a tube part in which a first lumen and a second lumen are formed, the first lumen being capable of being inserted by the endoscope, and the second lumen being capable of being inserted by the treatment tool; and
a distal end part in which a distal opening end of the first lumen and a distal opening end of the second lumen are formed apart from each other,
wherein the distal end part includes:
a distal end surface that forms the distal opening end of the first lumen such that a center of the distal opening end of the first lumen is located at a position apart from the predetermined central axis, and
a tapered surface that is inclined with respect to the predetermined central axis such that the tapered surface gradually approaches the first lumen from a proximal side toward a distal side in the direction of the predetermined central axis, at least part of the distal opening end of the second lumen being disposed on the tapered surface.

2. The overtube according to Claim 1, further comprising:
a first sealing part that is configured to airtightly seal the first lumen and airtightly seal a gap between the first lumen and the endoscope in a state where the endoscope is disposed within the first lumen; and
a second sealing part that is configured to airtightly seal the second lumen and airtightly seal a gap between the second lumen and the treatment tool in a state where the treatment tool is disposed within the second lumen.

3. The overtube according to Claim 1 or 2,
wherein the distal end part includes a cover part capable of airtightly sealing the distal end opening of the second lumen and capable of being opened and closed.

4. The overtube according to Claim 3,
wherein the cover part is capable of contacting the distal opening end of the second lumen from a distal side thereof and is movably coupled to the distal end part such that the distal opening end of the second lumen is opened by movement of the cover part toward more distal side than the distal opening end of the second lumen.

5. The overtube according to any one of Claims 1 to 4,
wherein the distal opening end of the first lumen is larger than a proximal opening end of the first lumen.

6. The overtube according to any one of Claims 1 to 5,
wherein the first lumen has a small-diameter part which is formed with an inner diameter smaller than an inner diameter of the distal opening end of the first lumen and through which the endoscope is capable of being inserted, at part of the first lumen more proximal than the distal opening end of the first lumen.

7. The overtube according to any one of Claims 1 to 6,
wherein at least part of the distal opening end of the second lumen is formed by the tapered surface.

8. The overtube according to any one of Claims 1 to 7, further comprising:
a movable mechanism that is configured to move the distal opening end of the second lumen with respect to the distal opening end of the first lumen.
